# EUROPEAN PATENT APPLICATION

(11) **EP 2 506 007 A2**
(43) Date of publication of application: **03.10.2012**
(21) Application number: 10833574.6
(22) Date of filing: 25.11.2010
(51) Int. Cl.: G01N 33/00, G01N 1/22

(54) **SYSTEM FOR MEASURING, USING A QMS, AN ABSOLUTE QUANTITY OF EACH COMPONENT OF A GAS**

(30) Priority: 26.11.2009 KR 20090115440
(71) Applicant: El Inc., Daejeon 305-343 (KR); Korea Research Institute Of Standards And Science, Daejeon 305-340 (KR)
(72) Inventor: YOON, Seok Rae, Anyang-si Gyeonggi-do 430-708 (KR); JOH, Seong Kweon, Seoul 121-240 (KR); PARK, Won Young, Daejeon 302-170 (KR); LEE, Keu Chan, Daejeon 302-814 (KR); KIM, Jin Seog, Daejeon 305-370 (KR); LEE, Jin Bok, Daejeon 305-755 (KR); MOON, Dong Min, Daejeon 305-729 (KR); KIM, Kwang Seob, Daejeon 305-755 (KR)
(74) Representative: Hocking, Adrian Niall
(86) International application number: PCT/KR2010/008388
(87) International publication number: WO 2011/065762

(57) **Abstract**

The present invention relates to a system for measuring an absolute quantity of each component of a gas using a QMS, more particularly to a system for measuring an absolute quantity of each component of a gas using a QMS, which is capable of performing not only a qualitative analysis but also a quantitative analysis in an accurate manner, using just a trace amount of gas introduced through a pinhole. According to the present invention, the productivity of products can be improved, and production management can be performed in an efficient manner, thereby improving industrial competitiveness.

## Description

### Technical Field

The present invention relates to a system for measuring an absolute quantity of each component of a gas using a QMS, more particularly to a system for measuring an absolute quantity of each component of a gas using a QMS, which is capable of performing not only a qualitative analysis but also a quantitative analysis in an accurate manner, using just a trace amount of gas introduced through a pinhole.

### Background Art

Generally, the basic principle of a gas analysis is PV = nRT (P = pressure, V = volume, n = mole number, R = gas constant, T = absolute temperature). Here, if other conditions are constant, P is proportional to n and P of each component is a partial pressure or a mole fraction thereof.

Thus, a unique sensitivity of each component is set through experiments using a standard material and a sample gas is measured by using the sensitivity, so that it draws the results of a qualitative analysis of each component.

In the event of a failure of a product having the gas using these principles, it is difficult to grasp the problem.

Therefore, it is necessary to grasp the cause of the failure by using the objective results of the gas analysis. Also, an appropriate modification on this is conducted, thereby improving the productivity thereof.

In order to grasp the above cause of the failure, even though a gas analysis is utilized through a conventional GC (Gas Chromatography) using a gas analysis equipment and a method thereof, where an advance information on the gas component materials and the partial pressure thereof are not existed, since it is necessary to perform a repetitive experiment so as to grasp the contents of the gas, long-term experiments and a large amount of sample gas are needed.

Also, where the contents of the gas sample are very complicated, sometimes, it cannot draw the result of the analysis.

Accordingly, in order to overcome the problem of the conventional GC analysis, in a NIST (U.S. National Institute of Standards and Technology), samples of 220,000 spectroscopic data analysis are provided, so that the results of the analysis and the spectrum data can be compared and contrasted with each other. However, it is difficult to dramatically decrease the period required for analysis.

Also, in a conventional gas analysis equipment and a method thereof using a FTIR (Fourier transformation infrared spectroscopy), it can draw the exact results of experiments on a polyatomic molecule. However, in case of a monoatomic molecule, there is a fatal defect in that it cannot conduct the analysis thereof.

### Disclosure

### Technical Problem

Therefore, the present invention has been made in view of the above-mentioned problems, and the object of the present invention is to provide a system for measuring an absolute quantity of each component of a gas using a QMS, which is capable of performing not only a qualitative analysis but also a quantitative analysis in an accurate manner, using just a trace amount of gas introduced through a pinhole.

Another object of the present invention is to provide a system for measuring an absolute quantity of each component of a gas using a QMS, in that it is possible to conduct several repetition measurements of the same samples by one time sampling through a simple analysis procedure, thereby remarkably decreasing the period of analysis and drawing the result of a quantitative analysis having a low uncertainty.

### Technical solution

In accordance with the present invention to achieve the object thereof, there is provided a system for measuring an absolute quantity of each component of a gas using a QMS comprising:
a main chamber 100 for storing a sample gas introduced through pinholes;
a QMS 200 for measuring a partial pressure of each component of the sample gas stored in the main chamber 100;
an ion gauge 400 for measuring a degree of vacuum of the main chamber 100 in real time connected to the main chamber 100;
a turbo pump pumping station 800 for discharging the sample gas stored in the main chamber 100 after the measuring of the QMS;
the pinholes 700 for inputting the sample gas into the QMS in a molecule flow condition;
a first valve 1000 formed at a pipe for connecting the pinholes 700 to a molecular chamber 300;
a baratron gauge 500 for measuring an absolute change of pressure inside the molecular chamber 300 connected to the molecular chamber 300;
the molecular chamber 300 for storing the sample gas entered through a second valve 1100 and a third valve 1200 therein;
the second and third valves 1100 and 1200 for forming a physical space so as to subdivide the sample gas in small quantity and supply it to the molecular chamber 300;
a fourth valve 1300 for transmitting the sample gas, which is stored in the molecular chamber 300, to a second turbo pump 810 connected to the molecular chamber 300;
the second turbo pump 810 for discharging the remaining gas remained in the molecular chamber 300 therethrough after completion of the measurement; and
a rotary pump 900 for rapidly treating the sample gas stored in a specimen chamber 600.

### Advantageous Effects

According to the system for measuring the absolute quantity of each component of the gas using the QMS according to the present invention, it is capable of performing not only the qualitative analysis but also the quantitative analysis in an accurate manner, using just the trace amount of gas introduced through a pinhole, so that the productivity of products can be improved and production management can be performed in an efficient manner, thereby improving industrial competitiveness.

### Brief Description of the Drawings

The foregoing and other objects, features and advantages of the present invention will become more apparent from the following detailed description when taken in conjunction with the accompanying drawings in which:
FIG. 1 is a schematic block diagram illustrating a system for measuring an absolute quantity of each component of a gas using a QMS according to the present invention; and
FIG. 2 is a flow chart illustrating a measuring method of a system for measuring an absolute quantity of each component of a gas using a QMS according to the present invention.

### <Descriptions on reference numbers for the major components in the drawings>

| | |
|---|---|
| 100: main chamber | 200: QMS |
| 300: molecular chamber | 400: ion gauge |
| 500: baratron gauge | 600: specimen chamber |
| 700: pinholes | |
| 800: turbo pump pumping station | |
| 810: second turbo pump | 900: rotary pump |
| 1000: first valve | 1100: second valve |
| 1200: third valve | 1300: fourth valve |

### Best Mode

### Mode for Invention

In accordance with the present invention to achieve the object thereof, there is provided a system for measuring an absolute quantity of each component of a gas using a QMS comprising:
a main chamber 100 for storing a sample gas introduced through pinholes;
a QMS 200 for measuring a partial pressure of each component of the sample gas stored in the main chamber 100;
an ion gauge 400 for measuring a degree of vacuum of the main chamber 100 in real time connected to the main chamber 100;
a turbo pump pumping station 800 for discharging the sample gas stored in the main chamber 100 after the measuring of the QMS;
the pinholes 700 for inputting the sample gas into the QMS in a molecule flow condition;
a first valve 1000 formed at a pipe for connecting the pinholes 700 to a molecular chamber 300;
a baratron gauge 500 for measuring an absolute change of pressure inside the molecular chamber 300 connected to the molecular chamber 300;
the molecular chamber 300 for storing the sample gas entered through a second valve 1100 and a third valve 1200 therein;
the second and third valves 1100 and 1200 for forming a physical space so as to subdivide the sample gas in small quantity and supply it to the molecular chamber 300;
a fourth valve 1300 for transmitting the sample gas, which is stored in the molecular chamber 300, to a second turbo pump 810 connected to the molecular chamber 300;
the second turbo pump 810 for discharging the remaining gas remained in the molecular chamber 300 therethrough after completion of the measurement; and
a rotary pump 900 for rapidly treating the sample gas stored in a specimen chamber 600.

At this time, the pinhole 700 is a perforated separation membrane formed at an inside of a pipe for connecting the main chamber to the first valve, a size of each pinhole is 10 to 500 µm, and the number thereof is 1 through 100.

Also, the physical space formed by the second and third valves 1100 and 1200 is 1.5L.

Moreover, the turbo pump pumping station 800 serves to maintain the degree of vacuum of the main chamber 100 below 10⁻⁷ mbar so as to flow the sample gas of the molecular chamber 300 into the main chamber 100 through the pinholes 700.

Furthermore, each size of the main chamber 100 and molecular chamber 300 is 50CC~10L.

Hereinafter, exemplary embodiments of the present invention will be described in detail with reference to the accompanying drawings.

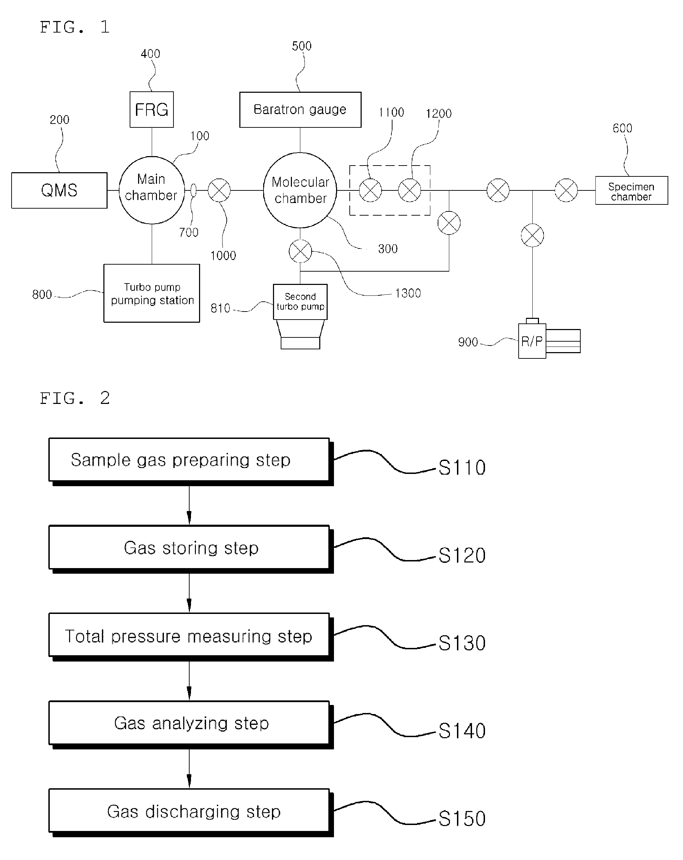

FIG. 1 is a schematic block diagram illustrating a system for measuring an absolute quantity of each component of a gas using a QMS according to the present invention.

As shown in FIG. 1, the system for measuring the absolute quantity of each component of the gas using the QMS according to the present invention includes:
a main chamber 100 for storing a sample gas introduced through pinholes;
a QMS 200 for measuring a partial pressure of each component of the sample gas stored in the main chamber 100;
an ion gauge 400 for measuring a degree of vacuum of the main chamber 100 in real time connected to the main chamber 100;
a turbo pump pumping station 800 for discharging the sample gas stored in the main chamber 100 after the measuring of the QMS;
the pinholes 700 for inputting the sample gas into the QMS in a molecule flow condition;
a first valve 1000 formed at a pipe for connecting the pinholes 700 to a molecular chamber 300;
a baratron gauge 500 for measuring an absolute change of pressure inside the molecular chamber 300 connected to the molecular chamber 300;
the molecular chamber 300 for storing the sample gas entered through a second valve 1100 and a third valve 1200 therein;
the second and third valves 1100 and 1200 for forming a physical space so as to subdivide the sample gas in small quantity and supply it to the molecular chamber 300;
a fourth valve 1300 for transmitting the sample gas, which is stored in the molecular chamber 300, to a second turbo pump 810 connected to the molecular chamber 300;
the second turbo pump 810 for discharging the remaining gas remained in the molecular chamber 300 therethrough after completion of the measurement; and
a rotary pump 900 for rapidly treating the sample gas stored in a specimen chamber 600.

More concretely, the system for measuring the absolute quantity of each component of the gas using the QMS according to the present invention includes the main chamber 100, the QMS 200, the ion gauge 400, the turbo pump pumping station 800, the pinholes 700, the first valve 1000, the baratron gauge 500, the molecular chamber 300, the second valve 100, the third valve 1200, the fourth valve 1300, the second turbo pump 810, the specimen chamber 600, and the rotary pump 900.

The main chamber 100 is connected to the QMS 200, the ion gauge 400, the pinholes 700, and the first turbo pump 800. The main chamber 100 serves to store the sample gas introduced through the pinholes 700. At this time, the main chamber 100 may be various shapes capable of storing the sample gas in a globular form or a cylindrical form.

The QMS 200 is the abbreviation of "Quantitative Mass System". In the prior art, it utilizes as only a qualitative analysis. However, in the present invention, the QMS 200 is combined with the main chamber 100, the ion gauge 400, the turbo pump pumping station 800, the pinholes 700, the first valve 1000, the baratron gauge 500, the molecular chamber 300, the second valve 1100, the third valve 1200, the fourth valve 1300, the second turbo pump 810, the specimen chamber 600, and the rotary pump 900, so that it can measure the partial pressure of each component of the sample gas too.

That is, as explained in the object of the present invention, it is capable of performing not only the qualitative analysis but also the quantitative analysis through the QMS 200.

The ion gauge 400 is connected to the main chamber 100 and serves to measure the degree of vacuum of the main chamber 100 in real time.

The turbo pump pumping station 800 serves to maintain the degree of vacuum of the main chamber 100 below 10⁻⁷ mbar and provide a high degree of vacuum to the main chamber 100 so as to flow the sample gas of the molecular chamber 300 into the main chamber 100 through the pinholes 700.

The pinholes 700 are formed at the inside of the pipe for connecting the main chamber 100 to the first valve 1000 so as to input the sample gas into the QMS 200 in a molecule flow condition. The size of each pinhole is 10 to 500 µm and the number thereof is 1 through 100. Also, it means a perforated separation membrane.

Thus, a certain number of molecules per hour pass through the pinholes and then, it can be injected into the main chamber 100 under an even pressure.

At this time, the number and the shape of the pinhole including the diameter thereof can be varied according to the measuring method or the kind of the sample.

The number and the diameter of the pinhole can be varied differently by measuring the pressure in advance through the ion gauge 400 according to the kind of the ample and the object of the measurement.

That is, where the injection pressure is higher than the expectations through the measurement of the pressure in advance, the number of the pinholes is decreased or the diameter thereof becomes narrow. Meanwhile, where the injection pressure is lower than the expectations, the number of the pinholes is increased or the diameter thereof is widened.

The first valve 1000 is formed at the pipe for connecting the pinholes 700 to the molecular chamber 300. If they are connected to each other, the first valve 1000 serves to supply the sample gas stored in the molecular chamber 300 to the pinholes 700.

The baratron gauge 500 is connected to the molecular chamber 300. It serves to measure the absolute change of pressure inside the molecular chamber 300. That is, in order to measure the sample gas, which is stored in the main chamber 100, in the QMS 200, if the small sample gas is discharged from the molecular chamber 300 to the main chamber 100, since the total pressure of the remaining gas, exclusive the discharged sample gas, is changed in the corresponding molecular chamber 300, it allows the baratron gauge 500 to measures it.

At this time, the baratron gauge 500 measures the absolute change of pressure inside the molecular chamber 300. Also, where all of the exit valves (first valve, second valve, third valve, and fourth valve) are opened, it severs to measure the entire pressure of the system.

That is, the baratron gauge 500 for measuring the absolute change of pressure in an accurate manner serves to collect the total pressure information of the injected gas during analysis thereof.

The second and third valves 1100 and 1200 serve to form the physical space capable of subdividing the sample gas in small quantity and supply it to the molecular chamber 300.

At this time, the physical space means any pipe including the second and third valves 1100 and 1200. Preferably, the pipe has any diameter and length for forming the physical space of about 1.5L.

In order to secure a MEAM FREE PATH (that is, the maximum distance capable of moving without collisions between molecules), the physical space must not be narrow. Also, if it is too wide, since the loss of sample is large, there is a problem in that the quality of the analysis data is deteriorated. Accordingly, it is preferred that it has the physical space of about 1.5L.

The fourth valve 1300 is connected to the molecular chamber 300 and serves to transmit the sample gas, which is stored in the molecular chamber 300, to the second turbo pump 810.

The second turbo pump 810 serves to remove the remaining gas remained in the molecular chamber 300 after end of the measurement.

The rotary pump 900 serves to rapidly treat the sample gas stored in the specimen chamber 600. After the sample gas stored in the specimen chamber 600 is rapidly discharged, it is maintained in a vacuum so as to inject another sample gas therein.

FIG. 2 is a flow chart illustrating a measuring method of a system for measuring an absolute quantity of each component of a gas using a QMS according to the present invention.

That is, the measuring method of the system for measuring the absolute quantity of each component of the gas using the QMS according to the present invention including:
a sample gas preparing step S110 for storing the sample gas in the specimen chamber 600;
a gas storing step S120 for storing the sample gas in the molecular chamber 300;
a total pressure measuring step S130 for injecting the stored sample gas into the main chamber 100 via the pinhole 700;
a gas analyzing step S140 for analyzing the injected gas under the adjusted pressure; and
a gas discharging step S150 for treating the remaining gas remained in the measurement apparatus after end of the sample analysis.

More concretely, after the sample gas is stored in the specimen chamber 600, the second and third valves 1100 and 1200 are opened, so that the gas is stored in the molecular chamber 300 (S120). Then, in order to flow the gas stored in the molecular chamber 300 into the main chamber 100, the first valve 1000 is opened, so that the total gas pressure of the molecular chamber 300 is very regularly decreased and the stored sample gas is injected into the main chamber 100 in a molecule flow condition through the pinholes 700 (S130), thereby performing the analysis (S140) through the QMS 200.

Also, after the result values are obtained according to the end of the analysis of the sample, the remaining gas is discharged through the first turbo pump and the rotary pump (S150).

Moreover, after the gas analysis is completed, the remaining gas is discharged through the turbo pump pumping station. Also, the exhaust line is connected to an outside or a laboratory exhaust line to be discharged to the atmosphere.

Generally, the amount of the sampling gas is several times through several hundred times that of one time measurement thereof.

Accordingly, the samples secured through one time sampling are stored in the interior space of the system exclusive the molecular chamber and then, they are subdivided when needed.

The measuring method of the present invention will be briefly described.

After the sample gas is stored in the specimen chamber 600 (S110), the sample gas is moved to the molecular chamber 300 by using the valves between molecular chamber 300 and the specimen chamber 600 to be stored (S120).

Thereafter, the baratron gauge 500 measures the total pressure of the sample gas, which is moved to the molecular chamber 300 and the absolute change of pressure inside the molecular chamber 300 before and after the injection to be recorded (S130).

Then, the first valve 1000 connected to the molecular chamber 300 is opened, so that the sample gas is moved to the main chamber 100 having the QMS 200 through the pinholes 700, thereby measuring the partial pressure of each component of the sample gas (S140). Here, the sensitivity of each component thereof is set by the standard material in advance.

Since the standard material is well-known in the art, a further description on this is omitted here.

After the gas analysis is completed, in order to treat the remaining gas existed inside the measuring apparatus, the remaining gas is discharged to the outside by means of the turbo pump pumping station (S150).

In conclusion, the case of conventional gas analysis system, it is impossible to perform the quantitative analysis having a low uncertainty and it is possible to restrictedly perform the comparative quantitative analysis only if the components are similar. However, in the present invention, it measures the total pressure of the sample gas in the baratron gauge and compares the partial pressure of each component with the standard material in the QMS, thereby knowing the mole% of each component.

Theoretically, it means mole% of each component of gas = partial pressure of gas = concentration. Also, it means the total pressure of gas = partial pressure a + partial pressure b + ... as the sum of the partial pressures of each component.

For example, it is possible to conduct an accurate quantitative analysis of the trace gas existed in a vacuum inside a lamp. Also, as though the limit of the measurement thereof depends on the amount of the gas, it is possible to usually measure up to a several ppm region.

Although several exemplary embodiments of the present invention have been described for illustrative purposes, those skilled in the art will appreciate that various modifications, additions and substitutions are possible, without departing from the scope and spirit of the invention as disclosed in the accompanying claims.

### Industrial Applicability

According to the system for measuring the absolute quantity of each component of the gas using the QMS according to the present invention, it is capable of performing not only the qualitative analysis but also the quantitative analysis in an accurate manner, using just the trace amount of gas introduced through a pinhole, so that the productivity of products can be improved and production management can be performed in an efficient manner, thereby improving industrial competitiveness. Accordingly, the system can be widely utilized in a semiconductor industry, a display industry, a CDM (Clean Development Mechanism) and so on.

## Claims

1. A system for measuring an absolute quantity of each component of a gas using a QMS comprising:
a main chamber 100 for storing a sample gas introduced through pinholes;
a QMS 200 for measuring a partial pressure of each component of the sample gas stored in the main chamber 100;
an ion gauge 400 for measuring a degree of vacuum of the main chamber 100 in real time connected to the main chamber 100;
a turbo pump pumping station 800 for discharging the sample gas stored in the main chamber 100 after the measuring of the QMS;
the pinholes 700 for inputting the sample gas into the QMS in a molecule flow condition;
a first valve 1000 formed at a pipe for connecting the pinholes 700 to a molecular chamber 300;
a baratron gauge 500 for measuring an absolute change of pressure inside the molecular chamber 300 connected to the molecular chamber 300;
the molecular chamber 300 for storing the sample gas entered through a second valve 1100 and a third valve 1200 therein;
the second and third valves 1100 and 1200 for forming a physical space so as to subdivide the sample gas in small quantity and supply it to the molecular chamber 300;
a fourth valve 1300 for transmitting the sample gas, which is stored in the molecular chamber 300, to a second turbo pump 810 connected to the molecular chamber 300;
the second turbo pump 810 for discharging the remaining gas remained in the molecular chamber 300 therethrough after completion of the measurement; and
a rotary pump 900 for rapidly treating the sample gas stored in a specimen chamber 600.

2. A system for measuring an absolute quantity of each component of a gas using a QMS as recited in claim 1, wherein the pinhole 700 is a perforated separation membrane formed at an inside of a pipe for connecting the main chamber to the first valve, a size of each pinhole is 10 to 500 µm, and the number thereof is 1 through 100.

3. A system for measuring an absolute quantity of each component of a gas using a QMS as recited in claim 1, wherein the physical space formed by the second and third valves 1100 and 1200 is 1.5L.

4. A system for measuring an absolute quantity of each component of a gas using a QMS as recited in claim 1, wherein the turbo pump pumping station 800 serves to maintain the degree of vacuum of the main chamber 100 below 10⁻⁷ mbar so as to flow the sample gas of the molecular chamber 300 into the main chamber 100 through the pinholes 700.

5. A system for measuring an absolute quantity of each component of a gas using a QMS as recited in claim 1, wherein each size of the main chamber 100 and molecular chamber 300 is 50CC~10L.
